(19) Europäisches Patentamt

European Patent Office

Office européen des brevets

(11) Veröffentlichungsnummer: **0 224 222 B1**

(12) **EUROPÄISCHE PATENTSCHRIFT**

(45) Veröffentlichungstag der Patentschrift: **13.05.92**

(21) Anmeldenummer: **86116209.7**

(22) Anmeldetag: **22.11.86**

(51) Int. Cl.⁵: **H01R 13/66**, H01R 31/06, H01R 13/453

(54) **Kabelverbinder.**

(30) Priorität: **25.11.85 DE 8533107 U**

(43) Veröffentlichungstag der Anmeldung:
**03.06.87 Patentblatt 87/23**

(45) Bekanntmachung des Hinweises auf die
Patenterteilung:
**13.05.92 Patentblatt 92/20**

(84) Benannte Vertragsstaaten:
**AT BE CH DE ES FR GB GR IT LI LU NL SE**

(56) Entgegenhaltungen:
**EP-A- 0 045 834**
**DE-A- 2 949 098**
**GB-A- 2 045 559**
**US-A- 4 280 507**

(73) Patentinhaber: **ARBO Medizin-Technologie
GmbH
Wendenstrasse 2-3
W-3300 Braunschweig(DE)**

(72) Erfinder: **Ding, Wolfgang
Moorhüttenweg 2e
W-3300 Braunschweig(DE)**
Erfinder: **Arnold, Werner
Georgstrasse 13
W-4053 Jüchen(DE)**

(74) Vertreter: **Lins, Edgar, Dipl.-Phys. et al
Patentanwälte Gramm + Lins Theodor-
Heuss-Strasse 2
W-3300 Braunschweig(DE)**

**Beschreibung**

Die Erfindung betrifft einen Kabelverbinder für eine Vielzahl von auf den menschlichen Körper aufgesetzten Meßelektroden zu einem Auswertungsgerät mit einer Mehrzahl von in einen Steckverbinder eingesteckten Einzelleitungen, die durch einen Schutzwiderstand geschützt sind.

Derartige Kabelverbinder sind bekannt. Die Einzelleitungen sind dabei im allgemeinen mit Bananensteckern an den Meßelektroden angeschlossen. Die anderen Enden weisen jeweils einen Stecker auf, die in einen n-Pol liegenden Steckverbinder einsteckbar sind, wobei n die Anzahl der Einzelleitungen ist. Der bekannte Steckverbinder ist fest mit einem Sammelkabel verbunden, an das an seinem anderen Ende ein Gerätestecker angeschweißt ist.

Da derartige Anordnungen auch für die Defibrillation verwendet werden, bei der dem Körper Stromschocks über die Elektroden zugeführt werden, müssen die Einzelleitungen Schutzwiderstände aufweisen. Diese sind in der bekannten Kabelverbinderanordnung in den Bananensteckern untergebracht. Werden Notreparaturen an dem Kabelverbinder erforderlich, kann es passieren, daß der Bananenstecker überbrückt wird, so daß der erforderliche Schutzwiderstand nicht wirksam ist. Darüber hinaus hat der bekannte Kabelverbinder den Nachteil, daß eine Beschädigung des Sammelkabels einen sehr kostspieligen Ersatz erfordert, da das Sammelkabel mit dem Steckverbinder und dem Gerätestecker ausgewechselt werden muß.

Aus GB-A-2045559 ist eine Steckverbindung zwischen einem EKG-Signale und Blutdruck-Signale simulierenden Generator und einem entsprechenden Anzeigegerät bekannt. Auf einem mehradrigen Kabel werden verschiedene Signale zum Anzeigegerät übertragen. In dem für das Anzeigegerät vorgesehenen Stecker sind einige der Leitungen mit einem Widerstand versehen und der größte Teil dieser Leitungen unter den Widerständen zu einem Anschlußkontakt zusammengefaßt. Die zu einem Kontakt zusammengefaßten Widerstände haben unterschiedliche Werte, um zu verschiedenen Blutdrucksignalgrößen gehörende Signale zu erzeugen. Ein einziges Kabel ist über einen Widerstand mit einer Anschlußklemme des Steckers verbunden. Dieser Widerstand hat die Aufgabe, einen vorbestimmten Spannungsabfall hervorzurufen. Alle Widerstände haben daher die Funktion, die Größe der simulierten Signale zu beeinflußen. Eine Schutzfunktion ist nicht offenbart. Der Steckverbinder weist auch keine eingesteckten Einzelleitungen auf sondern ist mit einem mehradrigen Kabel fest verbunden. Die Adern dieses Kabels sind nicht mit auf den menschlichen Körper aufgesetzten Meßelektroden verbunden, so daß eine Schutzfunktion für den menschlichen Körper auch nicht benötigt wird.

Der Erfindung liegt die Aufgabe zugrunde, einen Kabelverbinder der eingangs erwähnten Art so zu erstellen, daß einerseits eine höhere Flexibilität bei der Anwendung gewährleistet ist, andererseits die Sicherheit vor Überströmen besser gewährleistet ist.

Diese Aufgabe wird erfindungsgemäß dadurch gelöst, daß die Schutzwiderstände in dem Steckverbinder hinter den Steckbuchsen für die Einzelleitungen angeordnet sind und daß der Steckverbinder einen der Anzahl der Einzelleitungen entsprechenden vielpoligen Steckanschluß aufweist.

Bei dem erfindungsgemäßen Kabelverbinder sind die Schutzwiderstände in dem Steckverbinder untergebracht, der als separates Teil ausgebildet ist und in den sowohl die Einzelleitungen als auch ggf. das Sammelkabel einsteckbar ist. In besonderen Anwendungsfällen kann der Steckverbinder auch direkt in ein Auswertungsgerät eingesteckt werden, so daß das Sammelkabel gänzlich überflüssig ist.

Die Anordnung der Schutzwiderstände in dem Steckverbinder hat den Vorteil, daß die Widerstände in jedem Fall - unabhängig von der Art der verwendeten Einzelleitungen - wirksam bleiben. Darüber hinaus ist es nunmehr möglich, den Kabelverbinder mit Einzelleitungen zu verwenden, deren Bananenstecker als Schutzstecker ausgebildet sind, in dem sie am Umfang mit einem isolierenden, in das Bananensteckergehäuse gegen eine Rückstellfeder einschiebbaren Mantel umgeben sind. Die Spitze der Bananenstecker ist dabei mit einem isolierenden Abschlußstück versehen. Die nicht in die Elektroden eingesteckten Bananenstecker sind daher berührungssicher, wenn nicht der Mantel gegen die Rückstellfeder zurückgeschoben wird.

Da der Steckverbinder ein separates Teil ist, kann das ggf. benötigte Sammelkabel bei Beschädigungen relativ preiswert ausgewechselt werden, da hiervon der Steckverbinder nicht betroffen ist.

Die Erfindung soll im folgenden anhand eines in der Zeichnung dargestellten Ausführungsbeispiels näher erläutert werden. Es zeigen:

Figur 1: eine seitliche Ansicht eines erfindungsgemäßen Kabelverbinders;

Figur 2: eine Draufsicht auf die Einsteckseite des Kabelverbinders.

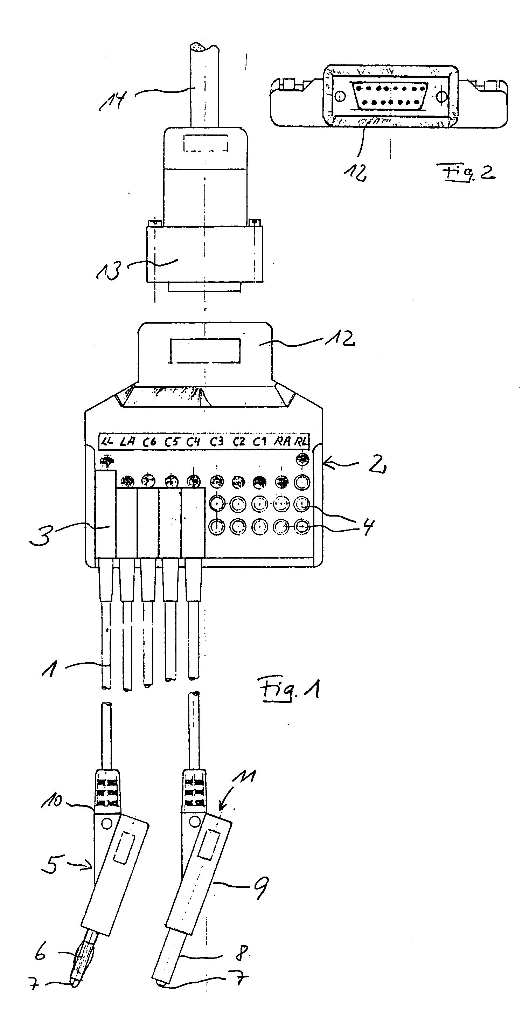

Figur 1 zeigt eine Vielzahl von Einzelleitungen 1, von denen zehn in einen Steckverbinder 2 mit Hilfe zwei oder mehrpoliger Stecker 3, deren Pole senkrecht zur Achse des Einzelkabels 1 steht, in entsprechende Buchsen 4 des Steckverbinders 2 einsteckbar sind. Die anderen Enden der Einzelleitungen 1 sind mit Sicherheits-Bananensteckern 5 versehen, von denen in der Figur 1 nur zwei dargestellt sind. Die Bananenstecker weisen einen üblichen Bananensteckkontakt 6 auf, der an seiner Spitze mit einem isolierenden Abschlußstück 7 versehen ist. Im nicht eingesteckten Zustand ist der Steckkontakt 6 durch einen Mantel 8 aus isolierendem Material, beispielsweise Kunststoff, abgedeckt. Der Mantel 8 ist gegen die Kraft einer Rückstellfeder in das Gehäuse 9 des Bananensteckers einschiebbar. Der in Figur 1 links dargestellte Bananenstecker 5 zeigt den Steckkontakt 6, nachdem der Mantel 8 in das Gehäuse 9 geschoben worden ist. Das Gehäuse 9 steht in einem spitzen Winkel zum Anschlußstück 10 des Einzelkabels 1 und ist an seinem hinteren Ende mit einer Buchse 11 versehen, in die ein Bananenstecker-kontakt 6 zu Prüfzwecken eingeschoben werden kann.

Der Steckverbinder 2 ist als separates Teil ausgebildet und weist auf einer Flachseite die tafelförmig angeordneten Buchsen 4 auf. Das Gehäuse des Steckverbinders 2 verjüngt sich zur anderen Seite hin und weist dort einen Steckanschluß 12 auf, dessen Kontaktzahl sich aus der Zahl der Einzelleitungen 1 ergibt. In den Steckanschluß ist beispielsweise ein Stecker 13 eines in dem darge-stellten Ausführungsbeispiel zehnadrigen Sammel-kabels 14 einschiebbar. Es ist jedoch auch möglich, den Steckanschluß 12 auf einen entsprechen-den Stecker eines Auswertungsgerätes aufzustek-ken.

Der Steckverbinder 2 weist in seinem Gehäuse für jede Einzelleitung 1 einen (nicht dargestellten) Schutzwiderstand auf, der somit nicht mehr in dem Bananenstecker 5 untergebracht ist. Dadurch erhält das Gehäuse 9 des Bananensteckers soviel freien Raum, daß der Mantel 8 vorgesehen sein kann, der in das Gehäuse 9 einschiebbar ist, wenn der Bananenstecker 5 auf eine Meßelektrode aufgesteckt wird. Dieser Platz hatte in dem Gehäuse 9 nicht zur Verfügung gestanden, wenn das Gehäuse 9 den Schutzwiderstand umfassen mußte.

Für einige, am Rand einsteckbare Stecker 3 weist der Steck verbinder 2 überzählige Buchsen 4 auf, die so angeschlossen sind, daß die zugehörigen Stecker 3 (LL und RL) auch in einer um 180° verdrehten Stellung einsteckbar sind. Dadurch können spezielle Elektroden (z. B. zur Anbringung jan den Beinen) u. U. ohne größere Kabelknickung angeschlossen werden.

## Patentansprüche

1. Kabelverbinder für eine Vielzahl von auf den menschlichen Körper aufgesetzten Meßelektroden zu einem Auswertungsgerät mit einer Mehrzahl von in einem Steckverbinder (2) eingesteckten Einzelleitungen (1), wobei die Einzelleitungen (1) durch einen Schutzwiderstand geschützt sind, dadurch **gekennzeichnet**, daß die Schutzwiderstände in dem Steckverbinder (2) hinter den Steckbuchsen (4) für die Einzelleitungen (1) angeordnet sind und daß der Steckverbinder (2) einen der Anzahl der Ein-zelleitungen entsprechenden vielpoligen Steck-anschluß (12) aufweist.

2. Verwendung eines Kabelverbinders nach Anspruch 1 mit in die Steckbuchsen (4) eingesteckten Einzelleitungen (1), deren freien Enden Bananenstecker (5) aufweisen, deren Steckkontakte (6) mit einem isolierenden, in das Bananensteckergehäuse (9) gegen eine Rückstellfeder einschiebbaren Mantel (8) umgeben sind und an ihrer Spitze ein isolierendes Abschlußteil (7) aufweisen.

## Claims

1. Cable connector for connecting a plurality of measuring electrodes placed on the human body to an evaluation device, having a plurality of individual lines (1) inserted in a plug connector (2), the individual lines (1) being protected by a protective resistor, characterized in that the protective resistors are arranged in the plug connector (2) behind the plug sockets (4) for the individual lines (1), and in that the plug connector (2) has a multi-pin plug connection (12) corresponding to the number of individual lines.

2. Use of a cable connector according to Claim 1, having individual lines (1) inserted in the plug sockets (4), the free ends of the individual lines (1) having banana plugs (5) whereof the plug contacts (6) are surrounded by an insulating casing (8) which may be pushed into the banana plug housing (9) in opposition to a return spring, which plug contacts (6) have at their tip an insulating terminal part (7).

## Revendications

1. Liaison de câblage destinée à relier à un appareil de traitement de données, plusieurs électrodes de mesure appliquées sur le corps humain, comprenant plusieurs conducteurs individuels (1) enfichés dans un connecteur (2), les conducteurs individuels (1) étant protégés par une résistance de protection, caractérisé en ce que les résistances de protection sont disposées dans le connecteur (2) derrière les fiches femelles (4) pour les conducteurs individuels (1), et en ce que le connecteur (2) comporte une prise de raccordement multipolaire (12) dont le nombre de pôles correspond au nombre de conducteurs individuels (1).

2. Utilisation d'une liaison câblage selon la revendication 1, avec des conducteurs individuels (1) enfichés dans les fiches femelles (4), les extrémités libres des conducteurs individuels (1) étant pourvues de fiches bananes (5) dont les fiches de contact (6) sont entourées par une enveloppe isolante (8), susceptible d'être repoussée à l'intérieur du boîtier (9) de la fiche banane à l'encontre d'un ressort de rappel, et présentent à leur pointe une pièce terminale isolante (7).

Fig. 2

Fig. 1